Europäisches Patentamt

European Patent Office

Office européen des brevets

(19)

(11) Numéro de publication: **0 107 579**
**A2**

# (12) DEMANDE DE BREVET EUROPEEN

(21) Numéro de dépôt: **83401999.4**

(22) Date de dépôt: **13.10.83**

(51) Int. Cl.³: **A 61 B 5/14**

(30) Priorité: **21.10.82 FR 8217634**

(43) Date de publication de la demande: **02.05.84**
**Bulletin 84/18**

(84) Etats contractants désignés: **AT BE CH DE GB IT LI LU
NL SE**

(71) Demandeur: **LE MATERIEL BIOMEDICAL Société à
Responsabilité Limitée dite:, 4 rue de Presbourg,
F-75116 Paris (FR)**

(72) Inventeur: **Lachenal, Jean-Michel, 25 avenue de la Paix,
F-94260 Fresnes (FR)**

(74) Mandataire: **Bonnetat, Christian et al, Cabinet PROPI
Conseils 23 rue de Léningrad, F-75008 Paris (FR)**

(54) **Système de prélèvement de liquide physiologique, tel que du sang et récipient pour un tel système.**

(57) Système de prélèvement de liquide physiologique, tel que
du sang, comportant des moyens d'aspiration et au moins un
récipient 1 pourvu d'une cloison transversale 3, disposée au
voisinage d'une extrémité ou obturant celle-ci et percée d'un
orifice 4, tandis qu'un organe de prélèvement 9 dudit liquide
est relié de façon amovible à l'autre extrémité du récipient.

Selon l'invention, ce système est caractérisé en ce que la
partie 2a dudit récipient comprise entre la cloison 3 et l'extrémité reliée audit organe de prélèvement 9 est conformée en cylindre pour un piston 5 solidaire d'une tige de piston 6 traversant ladite closion 3 par ledit orifice 4 et accessible à l'extérieur
dudit récipient 1, du côté opposé à l'organe de prélèvement 9.

ACTORUM AG

Système de prélèvement de liquide physiologique, tel que du sang et récipient pour un tel système.

La présente invention concerne un système de prélèvement de liquide physiologique, tel que du sang, et plus particulièrement un sytème permettant le prélèvement d'échantillons de faible volume, ainsi qu'un récipient pour un tel système.

De façon connue, le prélèvement de sang, notamment en vue de la constitution d'échantillons, est effectué à l'aide d'une seringue à aiguille, la traction du piston entraînant l'aspiration du sang dans le corps de la seringue.

Dans le cas où la quantité de sang prélevé est destinée à subir des opérations d'analyse ou de test, on doit, de façon connue, transférer le volume de sang contenu dans le corps de la seringue dans un ou plusieurs récipients selon le nombre d'opérations à effectuer.

Ce transfert entraîne des risques de contamination (par exemple en cas d'hépatite) de l'opérateur par le sang éventuellement infecté avec les conséquences graves que cette situation implique. Ce risque de contamination est d'autant plus élevé que le nombre d'analyses, donc de récipients différents, est plus grand.

En outre, ce transfert de sang prélevé, du corps de la seringue dans le ou les récipients, est susceptible de donner lieu à des erreurs ou à des difficultés de manipulations, notamment dans le cas de faibles volumes.

De plus, grâce aux perfectionnements des techniques et du matériel d'analyse sanguine, on peut maintenant obtenir des analyses et tests satisfaisants à partir de très faibles quantités de sang (quelques mm$^3$). Dans de nombreux cas, on peut prélever suffisamment de sang par la méthode capillaire (bout de doigt, oreille, etc...), qui met en oeuvre des tubes capillaires. Le contenu de ces tubes, après prélèvement, doit être transféré dans un autre récipient à des fins d'analyse. On conçoit qu'il est très difficile de prélever et transférer de façon précise de petits volumes de sang avec une seringue classique, et que cela est même impossible avec de très petits volumes.

Afin de remédier à ces inconvénients, on a déjà décrit dans la demande de brevet européen EP-A1-0 055 657 un système de prélèvement de sang (aussi bien capillaire que veineux) de mise en oeuvre simple, destiné à obtenir un volume de sang faible et correspondant exactement à la quantité désirée, les risques de contamination étant limités, du fait que le sang prélevé est introduit dans le récipient qui est directement utilisé pour réaliser les analyses et les tests envisagés.

Ce système de prélèvement de liquide physiologique, tel que du sang, comporte des moyens d'aspiration manuelle ou mécanique, par exemple une seringue, pourvus d'un cylindre et d'une aiguille, et est remarquable en ce qu'il comporte au moins un récipient tubulaire de faible diamètre susceptible d'être maintenu de façon amovible dans le prolongement du cylindre, du côté de l'aiguille, par des moyens de retenue portés par ledit cylindre, de manière que le fond dudit récipient soit traversé de façon étanche par ladite aiguille, ledit récipient étant pourvu d'un capuchon obturant son extrémité opposée à ladite aiguille et pourvu d'un organe de prélèvement dudit liquide.

On voit que, dans ce système, ladite aiguille sert de connexion entre les moyens d'aspiration et le récipient lorsque celui-ci est maintenu par les moyens de retenue et que l'aiguille traverse le fond dudit récipient, ce qui permet aux moyens d'aspiration d'aspirer l'air contenu dans le récipient et de forcer le liquide amené par l'organe de prélèvement à pénétrer dans ledit récipient, malgré le faible diamètre de celui-ci.

Ainsi, grâce à ce système, on évite le risque de contamination puisque le sang prélevé passe directement du patient dans le récipient qui sera utilisé pour effectuer les analyses ou tests nécessaires.

Lors de l'aspiration du sang par les moyens d'aspiration dans le cylindre, il est bien entendu nécessaire que le fond du récipient soit traversé par l'aiguille desdits moyens d'aspiration de manière étanche. Le fond doit en outre être étanche, lorsque ce dernier est désolidarisé des moyens d'aspiration pour effectuer les analyses ; autrement dit le sang contenu ne doit pas pouvoir sortir dudit récipient.

A cet effet, le fond du récipient comporte une cloison, en retrait de l'extrémité inférieure dudit récipient, qui est pourvue d'une lumière de diamètre suffisamment grand pour permettre le passage de l'aiguille des moyens d'aspiration et contre laquelle s'applique une membrane souple en un matériau élastique, tel que du caoutchouc, disposée du côté extérieur de la cloison et destinée à être percée par ladite aiguille, ladite membrane présentant une élasticité suffisante pour permettre, après enlèvement de l'aiguille, l'obturation de l'ouverture créée par l'enfoncement de ladite aiguille.

On obtient ainsi l'étanchéité aussi bien lorsque l'aiguille des moyens d'aspiration traverse la membrane, que lorsque ladite aiguille est retirée, lors de la désolidarisation du récipient et de ces moyens d'aspiration.

Cependant, ce système décrit dans le document EP-A1-0 055 657 s'est révélé d'un usage délicat. En effet, lors de l'actionnement des moyens d'aspiration mettant en dépression le récipient tubulaire, on s'est aperçu que ces moyens d'aspiration non seulement aspirent l'air du récipient tubulaire, permettant ainsi à celui-ci de se remplir de sang, mais encore le sang pénètre dans ledit récipient. Ceux-ci sont alors souillés et ne peuvent plus être utilisés pour un autre prélèvement. En cas d'actionnement brusque desdits moyens d'aspiration, il arrive même que, à la manière d'un arc électrique, il se forme un écoulement sanguin, direct et continu entre l'organe de prélèvement du capuchon monté sur le récipient et l'aiguille des moyens d'aspiration. La presque totalité du sang prélevé sur le donneur passe alors directement dans les moyens d'aspiration.

La présente invention a pour objet de remédier à cet inconvénient et de permettre le prélèvement direct de faibles volumes de sang.

A cette fin, selon la présente invention, le système de prélèvement de liquide physiologique, tel que du sang, comportant des moyens d'aspiration et au moins un récipient pourvu d'une cloison transversale, disposée au voisinage d'une extrémité ou obturant celle-ci et percée d'un orifice, tandis qu'un organe de prélèvement dudit liquide est relié de façon amovible à l'autre extrémité du récipient, est caractérisé en ce que la partie dudit récipient comprise entre la cloison et l'extrémité reliée audit organe de prélèvement est conformée en cylindre pour un piston disposé dans ladite partie et solidaire d'une tige de piston traversant ladite cloison par ledit orifice et accessible à l'extérieur dudit récipient, du côté opposé à l'organe de prélèvement.

Ainsi, en actionnant ladite tige de piston, il est possible de déplacer le piston à l'intérieur du récipient et d'aspirer à l'intérieur de celui-ci le sang prélevé par l'organe de prélèvement. Pendant le remplissage, le piston fait joint d'étanchéité par rapport à la paroi interne du récipient et le liquide ne peut passer de l'autre côté dudit piston. En fin d'aspiration, le piston se trouve appliqué contre la cloison interne et obture de façon étanche l'orifice de ladite cloison.

On voit qu'avec le système selon l'invention, on évite l'inconvénient mentionné ci-dessus à propos du système antérieur, notamment du fait que l'on supprime les moyens d'aspiration extérieurs, qui se trouvent maintenant incorporés au récipient lui-même.

De préférence, ladite tige de piston comporte une amorce de rupture de sorte que, en fin d'aspiration, on puisse facilement casser cette tige au voisinage de l'extrémité du récipient opposée à l'organe de prélèvement et que cette tige de piston ne soit pas gênante pour les utilisations et manipulations ultérieurs du récipient, dont le fond est alors formé par ladite cloison et le piston.

Par ailleurs, le système antérieur décrit ci-dessus présente l'inconvénient supplémentaire de ne permettre le prélèvement de sang que dans un seul récipient à la fois. Or, de nombreux tests exigent d'avoir à disposition plusieurs échantillons distincts. Par suite, le système antérieur oblige à prélever successivement plusieurs échantillons de sang sur le patient, c'est-à-dire à le piquer plusieurs fois avec l'aiguille servant généralement d'organe de prélèvement. Il est donc d'usage pénible pour le patient.

La présente invention permet de remédier à cet autre inconvénient, en prévoyant que, dans le système de prélèvement, on agence plusieurs récipients en parallèle sur un organe de prélèvement commun.

Les récipients montés en parallèle peuvent être de dimensions semblables ou différentes suivant les tests auxquels sont destinés les échantillons qu'ils contiennent.

De préférence, le système comporte alors un conduit souple par exemple, à une extrémité duquel est prévu l'organe de prélèvement, l'autre extrémité du conduit étant pourvue d'une pluralité de dérivations, sur chacune desquelles peut se fixer de façon amovible un récipient. Chaque dérivation peut être pourvue de moyens d'obturation, par exemple des pinces d'écrasement si les dérivations sont souples.

En aval desdites dérivations, ledit conduit peut être relié, par exemple de façon amovible, à une poche normale de prélèvement de sang d'une capacité de plusieurs centaines de $cm^3$. On remarquera qu'un tel système selon l'invention présente un avantage considérable sur les dispositifs de prélèvement à poche connus en usage pour les dons du sang. En effet, ces dispositifs connus comportent une poche de prélèvement reliée directement par un conduit souple à une aiguille ; aussi, le mode d'emploi de ces dispositifs connus consiste, après piqûre d'une veine du patient au moyen de l'aiguille, à laisser s'écouler le sang dans la poche jusqu'à ce que la quantité voulue soit atteinte, puis, l'aiguille restant en place dans la veine, on coupe le conduit pour isoler la poche et on recueille du conduit coupé, dans des tubes à essais, quelques échantillons de sang pour effectuer les vérifications nécessaires sur les caractéristiques du sang du donneur. On voit donc, qu'avec ces dispositifs connus, les échantillons de vérification ne peuvent être recueillis qu'après remplissage de la poche. Par suite, si le donneur éprouve un malaise devant entraîner l'arrêt immédiat du prélèvement, la partie de sang déjà prélevée est inutilisable et doit être jetée, puisqu'il sera impossible d'effectuer les tests de vérification. En revanche, avec le système selon l'invention, si le donneur est l'objet d'un malaise, la quantité de sang recueillie avant l'arrêt du prélèvement pourra être utilisée, puisque les échantillons nécessaires auront été recueillis en début de prélèvement, dans les récipients selon l'invention.

Les figures du dessin annexé feront bien comprendre comment l'invention peut être réalisée.

La figure 1 est une vue en coupe longitudinale d'un récipient pour système de prélèvement selon l'invention.

La figure 2 montre un exemple de réalisation du système selon l'invention.

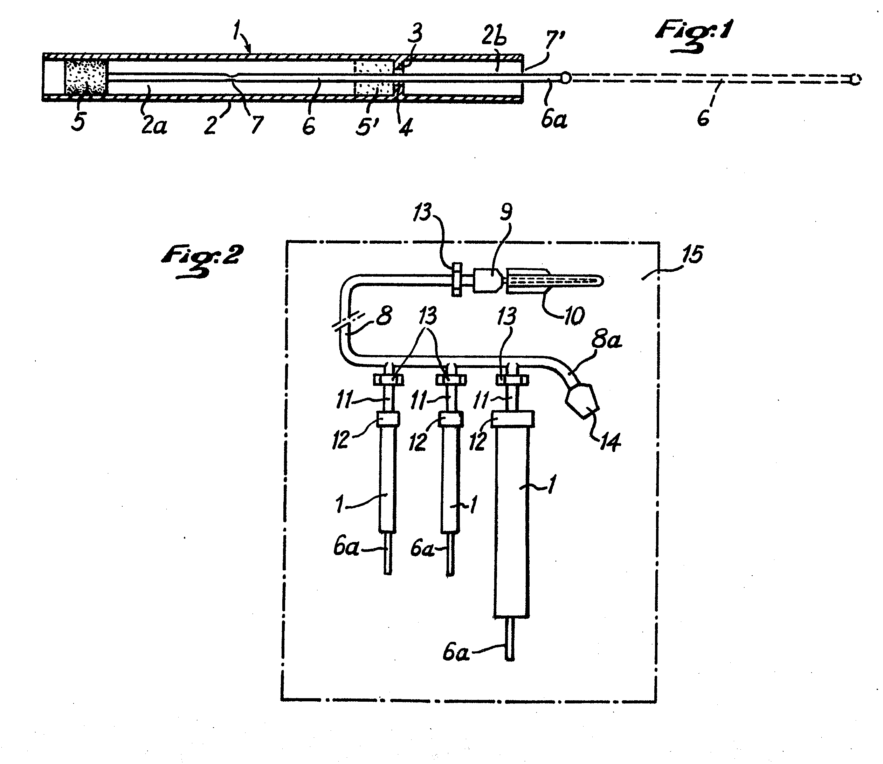

L'exemple de réalisation 1 du récipient selon l'invention pour le prélèvementt de sang, montré par la figure 1, comporte un corps tubulaire 2 pourvu d'une cloison transversale interne 3, percée d'un orifice traversant 4. Sur la figure 1, la cloison 3 est représentée en retrait par rapport à l'extrémité droite du corps tubulaire 2 de sorte que cette paroi sépare le volume interne du corps 2 en deux cavités 2a et 2b ; cependant, ceci n'est pas une obligation et la cloison 3 pourrait se trouver au niveau du bord de cette extrémité.

La plus importante des cavités, c'est-à-dire la cavité 2a, forme cylindre pour un piston 5, par exemple constitué d'un bloc d'élastomère. Le piston 5 est solidaire d'une tige de piston 6 qui traverse la cloison 3 par l'orifice 4 et dont l'extrémité 6a, opposée au piston 5, fait saillie à l'extérieur du corps tubulaire 2, après avoir traversé la cavité 2b, même lorsque le piston 5 occupe sa position la plus éloignée de la cloison 3.

La tige de piston 6 comporte une amorce de rupture 7 se trouvant au voisinage de l'extrémité droite du corps tubulaire 2 (position 7') lorsque la tige 6 est tirée à fond vers la droite, c'est-à-dire lorsque le piston 5 est en appui contre la cloison 3 (position 5'). Ainsi, il est possible après avoir amené le piston 5 en position 5' de briser la tige de piston 6 pour en éliminer la partie saillante qui risquerait d'être gênante.

Dans l'exemple de réalisation du système selon l'invention, montré par la figure 2, on a prévu plusieurs récipients 1 semblables à celui de la figure 1, mais de tailles différentes. Ce système comporte un conduit souple 8, à une extrémité duquel est fixé une aiguille 9, recouverte d'un protecteur 10.

A l'extrémité du conduit 8, opposée à l'aiguille 9, sont agencées une pluralité de dérivations 11 se terminant par des embouts 12, à chacun desquels est relié, par exemple par emboîtement, un récipient 1. Des pinces d'écrasement 13 sont prévues sur le conduit 8 au voisinage de l'aiguille 9 et sur les dérivations 11. En outre, en aval des dérivations 11, le conduit 8 se prolonge en 8a vers une poche de prélèvement de don de sang (non représentée) qui est soit soudée directement sur le prolongement 8a, soit reliée à celui-ci au moyen d'un raccord 14.

En vue de son stockage et de son transport, le système de la figure 2 , augmenté éventuellement d'une poche de prélèvement de don de sang, est enfermé dans une enveloppe stérile 15.

Pour utiliser le système selon l'invention, on le sort de l'enveloppe 15 et on introduit l'aiguille 9, dont le protecteur a été ôté, dans la veine d'un patient. En agissant de façon adéquate sur les pinces 13 et sur les pistons 5 des récipients 1 (par l'intermédiaire des tiges 6), on peut prélever dans lesdits récipients 1 et dans la poche de prélèvement non représentée les quantités désirées de sang.

Après prélèvement, on dégage les récipients 1 des embouts 12 pour les séparer de l'ensemble et on brise les tiges de pistons 6 en 7.

On obtient alors une pluralité d'échantillons de sang contenus chacun dans un récipient 1 dont la paroi 3 et le piston 5 (en appui sur celle-ci) forme le fond.

Il est avantageux que la position extrême des pistons 5 vers l'organe de prélèvement 9 soit limitée par une butée (non représentée), par exemple solidaire des raccords 12, pour maintenir lesdits pistons 5 en retrait de l'extrémité correspondante du tube associé. Ainsi, il est possible d'aspirer dans chaque cavité 2a un volume de sang déterminé, ce volume étant légèrement inférieur à la capacité maximale de ladite cavité, de sorte qu'il n'y a aucun risque de débordement.

REVENDICATIONS

1.- Système de prélèvement de liquide physiologique, tel que du sang, comportant des moyens d'aspiration et au moins un récipient 1 pourvu d'une cloison transversale 3, disposée au voisinage d'une extrémité ou obturant celle-ci et percée d'un orifice 4, tandis qu'un organe de prélèvement 9 dudit liquide est relié de façon amovible à l'autre extrémité du récipient, caractérisé en ce que la partie 2a dudit récipient comprise entre la cloison 3 et l'extrémité reliée audit organe de prélèvement 9 est conformée en cylindre pour un piston 5 solidaire d'une tige de piston 6 traversant ladite cloison 3 par ledit orifice 4 et accessible à l'extérieur dudit récipient 1, du côté opposé à l'organe de prélèvement 9.

2.- Système de prélèvement selon la revendication 1, caractérisé en ce que la tige de piston 6 comporte une amorce de rupture 7 permettant de supprimer facilement la partie extérieure de ladite tige 6 susceptible d'être gênante après que le piston 5 a été amené au contact de la cloison 3.

3.- Système de prélèvement selon l'une des revendications 1 ou 2, caractérisé en ce qu'il comporte plusieurs récipients 1 en parallèle sur un organe de prélèvement 9 commun.

4.- Système de prélèvement selon la revendication 3, caractérisé en ce que les récipients 1 sont de dimensions différentes.

5.- Système de prélèvement selon l'une des revendications 3 ou 4,
caractérisé en ce qu'il comporte un conduit 8 à une extrémité duquel est prévu l'organe de prélèvement 9, l'autre extrémité dudit conduit 8 étant pourvue d'une pluralité de dérivations 11, sur chacune desquelles peut se brancher de façon amovible un récipient 1.

6.- Système selon la revendication 5,
caractérisé en ce qu'il comporte une poche de prélèvement pour don du sang reliée audit conduit 8 en aval desdits récipients 1.

7.- Système selon la revendication 6,
caractérisé en ce qu'il forme une unité comportant une pluralité de récipients 1, un organe de prélèvement 9, une poche de prélèvement pour don du sang ou au moins des moyens 8a, 14 de raccordement à une telle poche et un conduit reliant ces différents composants.

8.- Récipient de prélèvement de liquide physiologique, tel que du sang, destiné au système de la revendication 1,
caractérisé en ce que la partie 2a dudit récipient comprise entre la cloison 3 et l'extrémité reliée audit organe de prélèvement 9 est conformée en cylindre pour un piston 5 solidaire d'une tige de piston 6 traversant ladite cloison 3 par ledit orifice 4 et accessible à l'extérieur dudit récipient 1, du côté opposé à l'organe de prélèvement 9.

9.- Récipient selon la revendication 8,

caractérisé en ce que la tige de piston 6 comporte une amorce de rupture 7 permettant de supprimer facilement la partie extérieure de ladite tige 6 susceptible d'être gênante après que le piston 5 a été amené au contact de la cloison 3.

Fig. 1

Fig. 2